# EUROPEAN PATENT SPECIFICATION

(11) **EP 0 522 956 B1**
(45) Date of publication and mention of the grant of the patent: **05.02.1997**
(21) Application number: 92401954.0
(22) Date of filing: 07.07.1992
(51) Int. Cl.: C07D 409/12, C07D 333/20, C07D 495/04

(54) **Preparation of 2-(2-thienyl) ethylamine and synthesis of thieno [3,2-C] pyridine derivatives therefrom**
Herstellung von 2-(2-Thienyl)Äthylamin und Synthese von Thieno(3,2-c)pyridinderivaten daraus
Préparation de 2-(2-thiényl)éthylamine, ainsi qu'une synthèse de dérivés de thiéno(3,2-c)pyridine correspondants

(30) Priority: 08.07.1991 US 726476
(43) Date of publication of application: 13.01.1993
(73) Proprietor: SANOFI, 75008 Paris (FR)
(72) Inventor: Harrington, Peter J., Louisville, Colorado 80027 (US); Sanchez, Ignacio H., Boulder, Colorado 80301 (US)
(74) Representative: Polus, Camille

(56) References cited:
- EP-A- 0 342 118
- EP-A- 0 439 404
- FR-A- 2 300 090
- LU-A- 74 545
- US-A- 4 128 561
- US-A- 4 997 945
- SYNTHESIS. no. 2, February 1990, STUTTGART DE pages 122 - 124 H. YINGLIN ET AL. 'A convenient Synthesis of Primary Amines using Sodium Diformylamide as A Modified Gabriel Reagent'

## Description

### BACKGROUND OF THE INVENTION

### FIELD OF THE INVENTION

The present invention relates to processes for the synthesis of thieno[3,2-c]pyridine derivatives, particularly ticlopidine, and specifically to an improved process for conversion of suitably functionalized derivatives of 2-(2-thienyl)ethanol to 2-(2-thienyl)ethylamine (TEA).

### BACKGROUND INFORMATION

Ticlopidine is a compound with desirable blood platelet aggregation inhibition qualities. Previous technology for the preparation of ticlopidine has entailed a low yielding, labor intensive process, employing certain potentially hazardous and expensive materials. The cost of preparing ticlopidine has, therefore, been high. It has been desired to provide improved synthetic process technology that allows for a hihher conversion, reduced labor usage, and the elimination of costly, potentially dangerous materials.

A variety of synthetic approaches to making ticlopidine have been described in the art, including improvements on the various steps of such synthetic processes, e.g., as described below.

Ticlopidine was first described by Castaigne in U.S. Patent No. 4,051,141, where the synthesis thereof was accomplished by condensation of a thieno[3,2-c]pyridine with o-chlorobenzyl chloride.

One desirable method of preparing ticlopidine calls for a N-substituted 2-(2'-thienyl)ethylamine as a key intermediate. The method involved conversion of 2-(2'-thienyl)ethanol to the corresponding sulfonate derivative and then to a secondary amine by reaction with o-chlorobenzylamine and cyclization to give ticlopidine free base, as described by Braye in U.S. Patent No. 4,127,580.

Previous methods for the preparation of 2-(2'-thienyl)ethylamine have suffered from several disadvantages, including low yields (e.g., where the reactions resulted in mixtures of undesirable side products) and high cost.

For example, Braye, U.S. Patent No. 4,128,561 describes a two-step process of making 2-(2'-thienyl)ethylamine by converting 2-(2'-thienyl)ethanol to N-2-(2'-thienyl)ethyl phthalimide, and then treating the phthalimide with diethylenetriamine to form the amine. Braye also describes the amination of 2-(2'-thienyl)alkyl sulfonates with ammonia at elevated temperature and pressure. Braye discloses problems encountered in the preparation of primary amines with ammonia, i.e., the tendency for the process to form secondary and tertiary amines as side products.

A process where 2-(2'-thienyl)ethyl bromide is treated with alcoholic ammonia at ordinary temperature for 8 days to produce 2-(2'-thienyl)ethylamine is described by Blicke, et al., J. Am. Chem. Soc., 64, 3, 477-480 (1942).

Other synthetic approaches to making 2-(2'-thienyl)ethylamine have been disclosed in the art, for example, as described below.

The reduction of 2-(2'-thienyl)acetamide with a hydride, e.g., lithium aluminum hydride to form 2-(2'-thienyl)ethylamine is described in Japanese Kokai J6 1221-184-A.

The electrochemical reduction of 2-(2'-nitrovinyl) thiophene to 2-(2'-aminoethyl)thiophene is described in UK Patent Application GB 2,013,196A.

The catalytic hydrogenation of thienylacetonitrile to form thienylethylamines is described in European Patent No. 274,324.

The reduction of 2-(2-nitrovinyl)thiophene to form 2-(2-thienyl)ethylamine employing a boron-containing reducing agent, preferably diborane is described in U.S. Patent No. 4,906,756.

The reduction of nitrovinyl thiophenes with a hydride, e.g., lithium aluminum hydride to form thiophene ethylamines is described in J. Heterocyclic Chem., 7, 1257-1268 (1970).

The reduction of arylacetonitriles with lithium aluminum hydride/aluminum chloride to form the corresponding 2-aryl-1-aminoethanes is described in Synthesis, 1, 40-42, (1987).

Synthesis n° 2 (1990) 122-124 describes a process for the preparation of primary amines of the formula RNH₂ starting from compounds of the formula RX in which X represents a leaving group such as a halogen atom or an arylsulfonyloxy radical.

US-4 997 945 teaches the preparation of ticlopidine starting from the carbamate salt of 2-(2'-thienyl)-ethylamine. It further results from this document that 2-(2'-thienyl)ethylamine may be prepared by reacting the corresponding halogen or alkyl- or arylsulfonate derivative with a metal salt of formula M-Y in the presence of ammonia gas.

The preparation of thieno[3,2-c]pyridine derivatives, particularly ticlopidine is complicated when the quantities to be prepared are on a large scale. The usefulness of a process for large scale production is gauged by several factors. For example, starting materials have to be available within the purity required; the process must be logistically efficient, e.g., the intermediates should not require isolation or purification (isolation or purification typically result in addition of steps and decrease in yield); and the procedure should return a yield sufficient to make the process commercially feasible. Shortcoming in any of the above parameters result in increased manufacturing costs, which impacts negatively on the desirability of the process. The present invention provides an efficient large scale process for the preparation of thieno[3,2-c]pyridine derivatives, particularly ticlopidine.

### SUMMARY OF THE INVENTION

One aspect of the present invention relates to novel compounds having the formula 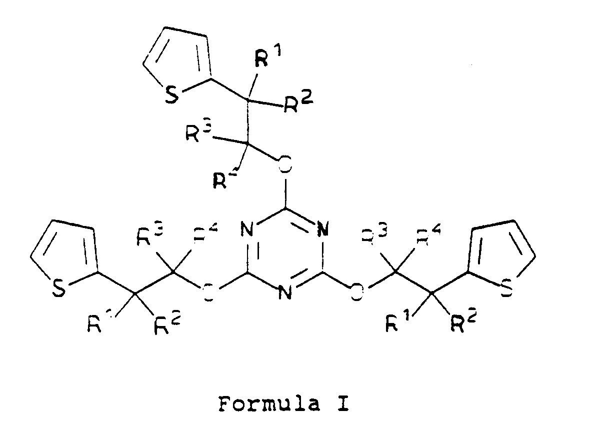 where:
- R¹, R², R³ and R⁴: are independently hydrogen, lower alkyl of one to six carbon atoms, aryl or substituted aryl.

Another aspect of the invention relates to a process for making the compounds of Formula I by contacting an alkyl metal salt, cyanuric halide and an optionally substituted 2-(2-thienyl)ethanol derivative.

Another aspect of the invention relates to novel compounds having the formula 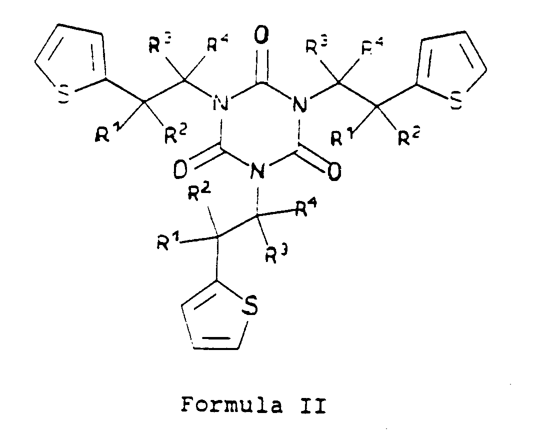 where:
- R¹, R², R³ and R⁴: are independently hydrogen, lower alkyl of one to six carbon atoms of one to six carbon atoms, aryl or substituted aryl.

Yet another aspect of the invention relates to a process for making the compounds of Formula II.

Still another aspect of the invention relates to a process for the synthesis of compounds of the following formula: 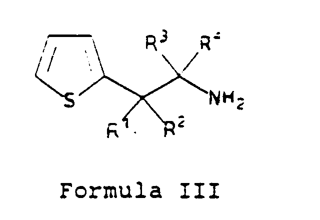 where:
- R¹, R², R³ and R⁴: are independently hydrogen, lower alkyl of one to six carbon atoms, aryl or substituted aryl;
from the compounds of Formula I.

In still another aspect, the invention relates to a process for the synthesis of compounds of the following formula: 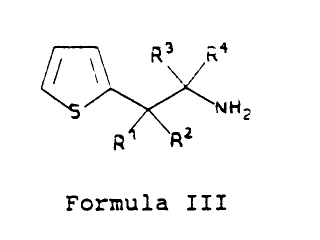
from the compounds of Formula II.

Still another aspect, the invention relates to a process for the synthesis of substituted benzyl thieno[3,2-c]pyridine derivatives of the following formula: 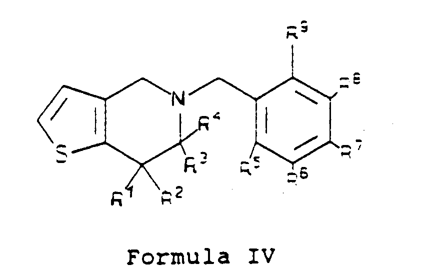 where:
- R¹, R², R³ and R⁴: are independently hydrogen or lower alkyl of one to six carbon atoms, aryl or substituted aryl; and
- R⁵, R⁶, R⁷, R⁸ and R⁹: are independently hydrogen, lower alkyl of one to six carbon atoms, alkoxy, acyl or halo; or a pharmaceutically acceptable salt thereof;
from the compounds of Formula I.

In still another aspect, the invention relates to a process for the synthesis of substituted benzyl thieno[3,2-c]pyridine derivatives of the following formula: 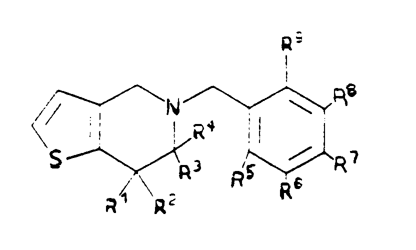 from the compounds of Formula II.

Still another aspect of the invention relates to a process for the synthesis of ticlopidine hydrochloride from the compounds of Formula I.

In still another aspect of the invention relates to a process for the synthesis of ticlopidine hydrochloride from the compounds of Formula II.

### DETAILED DESCRIPTION OF THE INVENTION

### DEFINITIONS AND GENERAL PARAMETERS

The following definitions are set forth to illustrate and define the meaning and scope of the various terms used to describe the invention herein.

A "pharmaceutically acceptable acid addition salt" of the thieno[3,2-c]pyridine derivatives may be any salt derived from an inorganic or organic acid, e.g., ticlopidine hydrochloride. The term "pharmaceutically acceptable anion" refers to the anion of such acid addition salts. The salt and/or the anion are chosen not to be biologically or otherwise undesirable. The anions are derived from inorganic acids, such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, salicylic acid, methanesulfonic acid, ethanesulfonic acid, p-toluenesulfonic acid and the like.

As used herein, the term "alkyl" refers to a cyclic, branched or straight chain monovalent alkyl radical of one to twenty-four carbon atoms.

As used herein, the term "lower alkyl" refers to a cyclic, branched or straight chain monovalent alkyl radical of one to six carbon atoms. This term is further exemplified by such radicals as methyl, ethyl, n-propyl, isopropyl, n-butyl, t-butyl, i-butyl, n-pentyl, cyclopentyl, n-hexyl, or cyclohexyl.

As used herein, the term "alkoxy" refers to the group O-R' where R' is alkyl.

As used herein, the term "lower alkoxy" refers to the group -O-R' where R' is lower alkyl.

As used herein, the term "acyl" refers to the group R'-C(O)- where R' is lower alkyl.

As used herein, the term "halo" refers to fluoro, bromo, chloro and iodo.

As used herein, the term "aryl" refers to a monovalent carbocyclic aromatic moiety, e.g., phenyl and naphthyl.

As used herein, the term "ketone" refers to a compound R-C(O)-R' where R and R' are the same or independently, branched or straight chain lower alkyl, cyclic alkyl or aryl (e.g., acetone, methylethyl ketone, diethyl ketone, cyclohexanone, methylphenylketone and diphenylketone).

As used herein, the terms "inert organic solvent" or "inert solvent" mean a solvent inert under the conditions of the reaction being described in conjunction therewith [including, for example, benzene, toluene, acetonitrile, tetrahydrofuran ("THF"), dimethylformamide ("DMF"), chloroform, methylene chloride (or dichloromethane), diethyl ether, methanol, ethanol, pyridine, dioxane, xylene, glyme and the like]. Unless specified to the contrary, the solvents used in the reactions of the present invention are inert organic solvents.

As used herein, the term "base" refers to compounds that will remove or accept a proton(s) from another molecule, such as, N-methylmorpholine, N-ethylmorpholine, pyridine, dialkylanilines, diisopropylcyclohexylamine, sodium hydroxide, potassium hydroxide, ammonium hydroxide, sodium bicarbonate or potassium bicarbonate.

As used herein, the term "organic base" refers to hydrocarbon bases such as, N-methylmorpholine, N-ethylmorpholine, pyridine, dialkylanilines or diisopropylcyclohexylamine.

As used herein, "leaving group" means an atom or a group, charged or uncharged, that is detachable from another atom in what is considered to be the residual or main part of a molecule, including such leaving groups as, halo, alkyl sulfonates (e.g., methanesulfonate), aryl sulfonates, phosphates, sulfonic acid and sulfonic acid salts.

For the compounds of the instant invention containing a 2-(2-thienyl)ethyl moiety (e.g., the compounds of Formula III) the following numbering system will be used for naming said compounds.

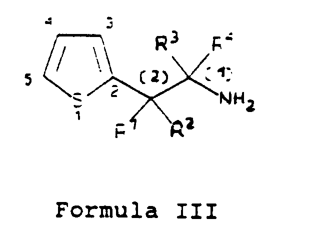

Some representative compounds are named in the following examples.

The compound of Formula III where R¹, R², R³ and R⁴ are hydrogen, can be named: 2-(2-thienyl)ethylamine.

The compound of Formula III where R¹ is methyl, R², R³ and R⁴ are hydrogen, can be named: 2-methyl-2-(2-thienyl)ethylamine.

The compound of Formula III where R³ is methyl, R¹, R² and R⁴ are hydrogen, can be named: 1-methyl-2-(2-thienyl)ethylamine.

The compound of Formula III where R¹ is methyl, R³ is benzyl, R² and R⁴ are hydrogen, can be named: 1-benzyl-2-methyl-2-(2-thienyl)ethylamine.

The compound of Formula III where R² is *p*-toluenyl, R⁴ is methyl, R¹ and R³ are hydrogen, can be named: 1-methyl-2-*p*-toluenyl-2-(2-thienyl)ethylamine.

For the compounds of the instant invention containing a cyanurate ring moiety (e.g., the compounds of Formula I) the following numbering system will be used for naming said compounds.

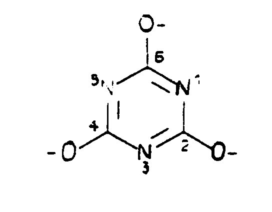

Some representative compounds are named in the following examples.

The compound of Formula I where R¹, R², R³ and R⁴ are hydrogen, can be named: 2,4,6-*tris*-(2-(2-thienyl)ethyl)cyanurate.

The compound of Formula I where R¹ is methyl, R², R³ and R⁴ are hydrogen, can be named: 2,4,6-*tris*-(2-methyl-2-(2-thienyl)ethyl)cyanurate.

The compound of Formula I where R³ is methyl, R¹, R² and R⁴ are hydrogen, can be named: 2,4,6-*tris*-(1-methyl-2-(2-thienyl)ethyl)cyanurate.

The compound of Formula I where R¹ is methyl, R³ is benzyl, R² and R⁴ are hydrogen, can be named: 2,4,6-*tris*-(1-benzyl-2-methyl-2-(2-thienyl)ethyl)cyanurate.

The compound of Formula I where R² is *p*-toluenyl, R⁴ is methyl, R¹ and R³ are hydrogen, can be named: 2,4,6-*tris*-(1-methyl-2-*p*-toluenyl-2-(2-thienyl)ethyl)cyanurate.

For the compounds of the instant invention containing a isocyanurate ring moiety (e.g., the compounds of Formula II) the following numbering system will be used for naming said compounds.

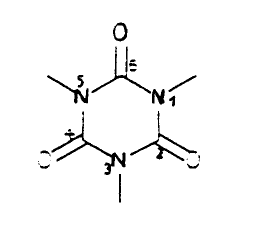

Some representative compounds are named in the following examples.

The compound of Formula II where R¹, R², R³ and R⁴ are hydrogen, can be named: 1,3,5-*tris*-(2-(2-thienyl)ethyl)isocyanurate.

The compound of Formula II where R¹ is methyl, R², R³ and R⁴ are hydrogen, can be named: 1,3,5-*tris*-(2-methyl-2-(2-thienyl)ethyl)isocyanurate.

The compound of Formula II where R³ is methyl, R¹, R² and R⁴ are hydrogen, can be named: 1,3,5-*tris*-(1-methyl-2-(2-thienyl)ethyl)isocyanurate.

The compound of Formula II where R¹ is methyl, R³ is benzyl, R² and R⁴ are hydrogen, can be named: 1,3,5-*tris*-(1-benzyl-2-methyl-2-(2-thienyl)ethyl)isocyanurate.

The compound of Formula II where R² is *p*-toluenyl, R⁴ is methyl, R¹ and R³ are hydrogen, can be named: 1,3,5-*tris*-(1-methyl-2-*p*-toluenyl-2-(2-thienyl)ethyl)isocyanurate.

For the compounds of the instant invention containing a thienopyridine moiety (e.g., the compounds of Formula IV) the following numbering system will be used for naming said compounds.

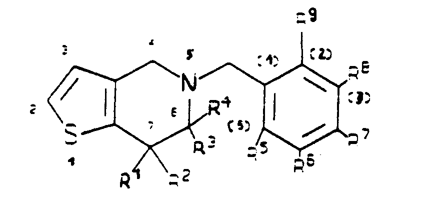

Some representative compounds are named in the following examples.

The compound of Formula IV where R⁵ is chloro; and R¹, R², R³, R⁴, R⁶, R⁷, R⁸ and R⁹ are hydrogen, (i.e., ticlopidine) can be named: 5-(2-chlorobenzyl)-4,5,6,7-tetrahydrothieno[3,2-c]pyridine or N-(2-chlorobenzyl)-4,5,6,7-tetrahydrothieno[3,2-c]pyridine, also known as ticlopidine.

The compound of Formula IV where R¹ is methyl; R⁵, and R⁹ are chloro; and R², R³, R⁴, R⁶, R⁷, and R⁸ are hydrogen, can be named: 5-(2,6-dichlorobenzyl)-4,5,6,7-tetrahydro-7-methyl-thieno[3,2-c]pyridine or N-(2,6-dichlorobenzyl)-4,5,6,7-tetrahydro-7-methyl-thieno[3,2-c]pyridine.

The compound of Formula IV where R³ is methyl; R⁷ is methoxy; R⁵ is chloro and R¹, R², R⁴ and R⁶ are hydrogen, can be named: 5-(2-chloro-4-methoxybenzyl)-4,5,6,7-tetrahydro-6-methyl-thieno[3,2-c]pyridine or N-(2-chloro-4-methoxybenzyl)-4,5,6,7-tetrahydro-6-methyl-thieno[3,2-c]pyridine.

The compound of Formula IV where R³ is ethyl; R⁵, R⁷ and R⁹ are chloro; and R¹, R², R⁴, R⁶ and R⁸ are hydrogen, can be named: 5-(2,4,6-trichlorobenzyl)-4,5,6,7-tetrahydro-6-ethyl-thieno[3,2-c]pyridine or N-(2,4,6-trichlorobenzyl)-4,5,6,7-tetrahydro-6-ethyl-thieno[3,2-c]pyridine.

Unless specified to the contrary, the reactions described herein take place at atmospheric pressure over a temperature range from about 10°C to about 100°C, more preferably from about 10°C to about 50°C, and most preferably at about room (or "ambient") temperature, e.g., about 20°C-30°C.

Isolation and purification of the compounds and intermediates described herein can be effected, if desired, by any suitable separation or purification procedure such as, for example, filtration, extraction, crystallization, column chromatography, thin-layer chromatography or thick-layer chromatography, or a combination of these procedures. Specific illustrations of suitable separation and isolation procedures can be had by reference to the examples hereinbelow. However, other equivalent separation or isolation procedures can, of course, also be used.

As used herein, the term "treatment" or "treating" means any treatment of a disease in a mammal, including:
(i) preventing the disease, that is, causing the clinical symptoms of the disease not to develop;
(ii) inhibiting the disease, that is, arresting the development of clinical symptoms; and/or
(iii) relieving the disease, that is, causing the regression of clinical symptoms.

### SYNTHESIS OF THE COMPOUNDS OF FORMULAE I, II, III AND IV

The compounds of Formulae I, II, III and IV are synthesized as described with reference to Reaction Schemes A, B and C. As used in the reaction schemes, R¹, R², R³ and R⁴ are independently hydrogen, lower alkyl of one to six carbon atoms, aryl or substituted aryl; and R⁵, R⁶, R⁷, R⁸ and R⁹ are independently hydrogen, lower alkyl of one to six carbon atoms, alkoxy, acyl or halo. The desired product from each preparation can be isolated and purified, however, preferably the unpurified product is taken directly to the next preparation.

Reaction Scheme A illustrates the conversion of suitable derivatives of 2-(2-thienyl)ethanol through the intermediate compounds of Formula I and II to form the corresponding 2-(2-thienyl)ethylamine derivatives (Formula III).

Reaction Scheme B illustrates another conversion of suitable derivatives of 2-(2-thienyl)ethanol to form the corresponding 2-(2-thienyl)ethylamine derivatives (Formula III).

Reaction Scheme C illustrates the conversion of suitable 2-(2-thienyl)ethylamine derivatives (Formula III) to the desired substituted thieno[3,2-c]pyridine derivative (Formula IV). 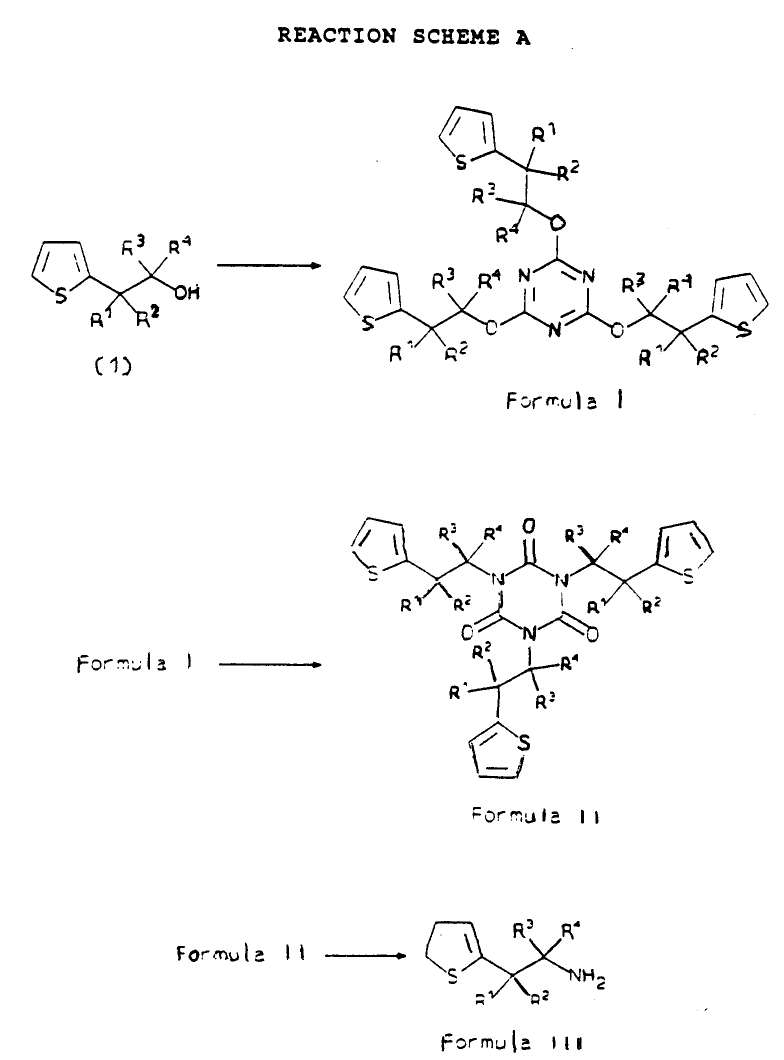 Referring to Reaction Scheme A, the starting materials (the compounds of Formula 1) are substituted 2-(2-thienyl)ethanol compounds, such as 2-methyl-2-(2-thienyl)ethanol, 1-methyl-2-(2-thienyl)ethanol, 1-benzyl-2-methyl-2-(2-thienyl)ethanol and 1-methyl-2-*p*-toluenyl-2-(2-thienyl)ethanol. Many of these materials are available commercially from such suppliers as, Aldrich Chemical Company or Henley Chemicals, 50 Chestnut Ridge Road, Montvale, New Jersey, 07645. Alternatively, they can be easily prepared according to procedures that are well known to the art and published in the literature, such as, Goldfarb, Y.L.; Kirmalova, M.L. *J. Gen. Chem. USSR* 1955, 25, 1321; *Chem Abstr.* 1956, 50, 6422h.

### PREPARATION OF THE COMPOUNDS OF FORMULA I

A substituted 2-(2-thienyl)ethanol compound (a compound of Formula 1) is dissolved in a solvent or a mixture of solvents (e.g., a polar solvent such as tetrahydrofuran, diethyl ether, dioxane, or toluene, preferably tetrahydrofuran). About 1 molar equivalent of *n*-alkyllithium (e.g., *n*-butyllithium, sodium hydride, lithium diisopropyl amide, or *sec*-butyllithium preferably *n*-butyllithium), is added to the solution in a gradual manner, preferably dropwise, over a period of about 2 hours, preferably about 49 minutes at a temperature in the range of about 0°C to -150°C, preferably about -70°C. The solution is stirred for about 5 to 30 minutes, preferably about 15 minutes, at a temperature in the range of about 0°C to -60°C, preferably about -30°C. About 0.33 molar equivalents of cyanuric halide (2,4,6-trihalo-1,3,5-triazine) is added to the solution in a gradual manner, preferably dropwise, over a period of about 2 to 15 minutes, preferably about 7 minutes, at a temperature in the range of 0 to -70°C, preferably about -20°C to -30°C. After the addition is completed, the solution is allowed to warm to about 25°C and stirred for about 12 to 24 hours, preferably about 18 hours. The desired O-substituted cyanurate compounds (Formula I) can be purified by removal of solvents and extraction followed by recrystallization, however, preferably the unpurified compounds are taken directly to the next preparation.

### PREPARATION OF THE COMPOUNDS OF FORMULA II

A O-substituted cyanurate compound (Formula I) and about 0.30 molar equivalents of a tetraalkylphosphonium halide (e.g., tetrabutylphosphonium bromide, tetraphenylphosphonium iodine, tetrabutylammonium iodine, preferably, tetrabutylphosphonium bromide) is heated in a sealed vessel at a temperature in the range of about 75°C to 175°C, preferably about 125°C for a period of about 24 to 64 hours, preferably about 44 hours. The reaction mixture is allowed to cool to about 25°C and the residual solid is slurried in an organic solvent (e.g., ethyl ether) and filtered. The mother liquor solvent is removed. The desired N-substituted isocyanurate compounds (Formula II) are purified by chromatography (e.g., radial chromatography) and recrystallization or can be taken to the next preparation without further purification.

### PREPARATION OF THE COMPOUNDS OF FORMULA III

A N-substituted isocyanurate compound (Formula II) and about 2.50 molar equivalents of a strong base (e.g., sodium hydroxide, potassium hydroxide or ammonium hydroxide, preferably sodium hydroxide) are dissolved in a polar solvent (e.g., butanol, tetrahydrofuran, ethanol, preferably butanol) and refluxed for a period of about 48 to 96 hours, preferably about 72 hours. The desired substituted 2-(2-thienyl)ethylamine compounds (Formula III) are isolated by removal of solvents and extraction and purified by distillation under reduced pressure, however, preferably the compounds are taken directly to the preparation without further purification.

### PREPARATION OF THE SALTS OF THE COMPOUNDS OF FORMULA III

A substituted 2-(2-thienyl)ethylamine compound (Formula III) is dissolved in about 3.7 liters/molar equivalent of a solvent (e.g., ethyl ether, diethyl ether, tetrahydrofuran, preferably ethyl ether). Gaseous acid (e.g., hydrogen chloride, hydrogen sulfide and hydrogen bromide, preferably, hydrogen chloride) is bubbled through the solution until the solution is thick with precipitate. The desired substituted 2-(2-thienyl)ethylamine salt compounds are washed with a solvent (e.g., ethyl ether, diethyl ether, tetrahydrofuran, preferably ethyl ether) and taken to the next preparation without further purification.

### ALTERNATE PREPARATION OF THE COMPOUNDS OF FORMULA III

Reaction Scheme B illustrates the conversion of suitable derivatives of 2-(2-thienyl)ethanol through the intermediate amide/imide compounds (compounds of Formulas 3 and 4) to form the corresponding 2-(2-thienyl)ethylamine derivatives (Formula III).

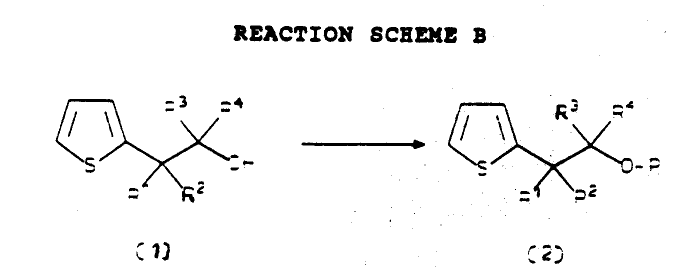

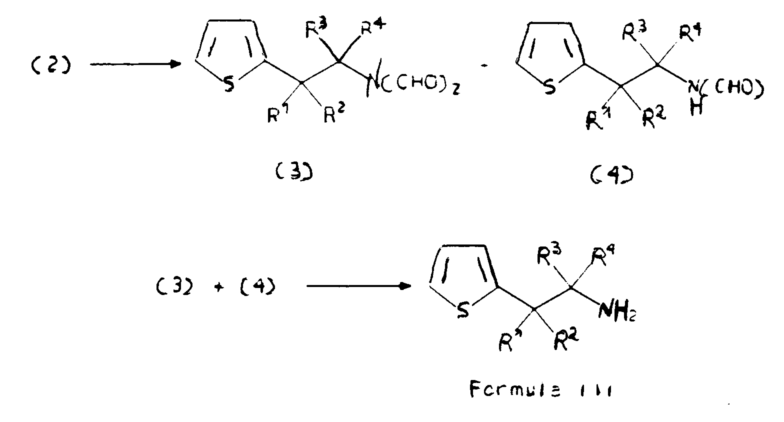

### PREPARATION OF THE COMPOUNDS OF FORMULA 2, WHERE P IS A PROTECTING GROUP.

Protected substituted 2-(2-thienyl)ethanol compounds (Formula 2) are prepared following procedures known in the art [e.g., arylsulfonates and alkylsulfonates (such as benzenesulfonate and methanesulfonate) are prepared according to the procedure of Braye U.S. Patent No. 4,127,580].

### PREPARATION OF THE COMPOUNDS OF FORMULAE 3 AND 4

Sodium metal is added to a dry solvent (e.g., methanol or ethanol, preferably methanol) and the mixture is refluxed to complete the reaction of the metal. The solution is cooled to about 25°C and 1.5 molar equivalents of formamide is added over a period of about 1 to 3 minutes, preferably about 2 minutes. The solution is stirred for a period of about 30 to 90 minutes, preferably about 1 hour. The solution is concentrated by removal of the solvent to yield solid sodium diformylimide.

A protected substituted 2-(2-thienyl)ethanol compound (Formula 2), about 1.18 molar equivalents of sodium diformylamide and about 0.63 Liters/molar equivalent of an aprotic polar solvent, such as, dimethylformamide, dimethylsulfoxide or N-methyl-2-pyrrolidinone, preferably dimethylformamide are heated at a temperature in the range of 70°C to 150°C, preferably about 110°C for a period of about 3 to 6 hours, preferably about 4.5 hours. The solvent is removed and the desired amide/imide compounds (Formulae 3 and 4, respectively) are purified and isolated by washing and extraction with solvent and chromatography (e.g., radial chromatography or the like), however, preferably the desired compounds are taken without further purification to the next preparation.

### PREPARATION OF THE COMPOUNDS OF FORMULA III

A mixture of the substituted 2-(2-thienyl)ethylimide and substituted 2-(2-thienyl)ethylamide (Formulae 3 and 4, respectively) is refluxed in a concentrated acid (e.g., hydrochloric acid, sulfuric acid or nitric acid, preferably 6N hydrochloric acid) for a period of about 1 to 3 hours, preferably about 2 hours. The solution is cooled to a temperature in the range of about 15 to 35°C, preferably about 25°C. The desired substituted 2-(2-thienyl)ethylamines (Formula III) are isolated by treatment with base, preferably sodium hydroxide, and purified by removal of the solvents followed by extraction with a suitable solvent (e.g., ethyl ether, diethyl ether, hexane, tetrahydrofuran, preferably ethyl ether) or preferably are taken without further purification to the next preparation.

### PREPARATION OF THE SALTS OF THE COMPOUNDS OF FORMULA III

The salts of the compounds of Formula III (that are prepared following Reaction Scheme B) can be made using the same procedure previously described for Reaction Scheme A.

Reaction Scheme C illustrates the conversion of suitable derivatives of 2-(2-thienyl)ethylamine (Formula III) to form the corresponding thieno[3,2-c]-pyridine derivatives (Formula IV), as described in U.S. Patent No. 4,997,945. R¹, R², R³ and are as previously defined in Reaction Schemes A and B. R⁴, R⁵, R⁶, R⁷, R⁸ and R⁹ are independently hydrogen, lower alkyl of one to six carbon atoms, alkoxy, acyl or halo; and X is chloro or bromo.

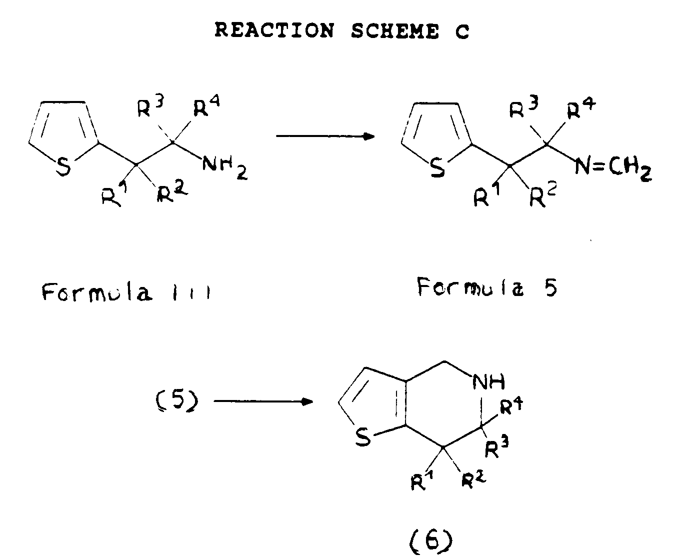

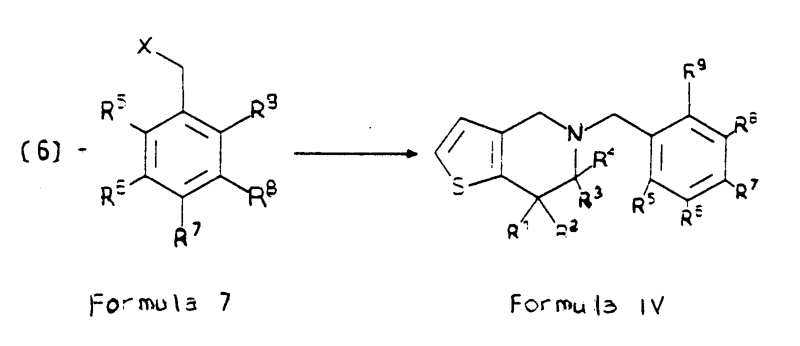

### PREPARATION OF THE COMPOUNDS OF FORMULA 5

A substituted 2-(2-thienyl)ethylamine (Formula III) and about 1 molar equivalents of 36.5% wt aqueous formaldehyde and about 2.36 molar equivalent of a solvent (e.g., water, ethanol, methanol or preferably toluene) are mixed. The suspension is heated at a temperature in the range of about 30°C to 130°C, preferably about 70°C to 95°C for a period of about 2 to 6 hours, preferably about 4 hours. The desired substituted formimines of 2-(2-thienyl)ethylamine (Formula 5) can be purified and isolated by removal of the solvents followed by extraction or the like. The formimines (Formula 5) can be isolated as monomers or as trimers [e.g., where R¹, R², R³ and R⁴ are hydrogen, N-formylidene-2-(2-thienyl)ethylamine or 1,3,5-*tris*-(2-(2-thienyl)ethyl)-hexahydro-*s*-triazine, respectively]. 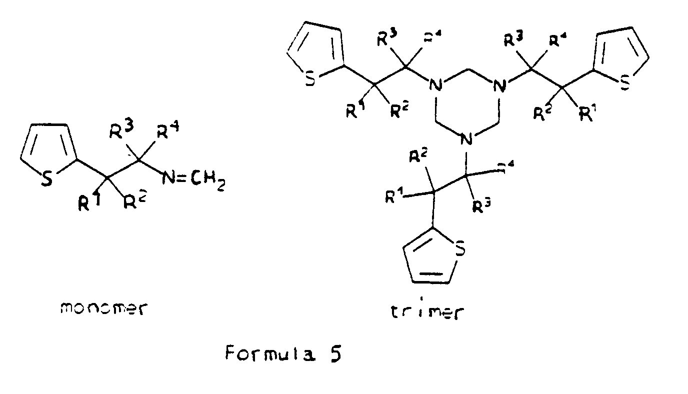
Preferably, the formimine is taken without purification directly to the next step.

### PREPARATION OF THE COMPOUNDS OF FORMULA 6

A formimine of a substituted 2-(2-thienyl)ethylamine, monomer or trimer, (Formula 5) is shaken with a dilute solution of an aqueous acid (such as hydrochloric acid or sulfuric acid), preferably the formimine is dissolved in an organic solvent (e.g., tetrahydrofuran or toluene, preferably tetrahydrofuran) and stirred with an acid solution (e.g., hydrogen chloride (gas) in tetrahydrofuran) for a period of about 2 to 6 hours, preferably about 4 hours at a temperature in the range of about -10°C to 20°C, preferably about 0°C to 15°C. The desired substituted 4,5,6,7-tetrahydrothieno[3,2-c]pyridines (Formula 6) can be isolated and purified by washing and removal of the solvents. However, preferably these compounds are taken directly to the next step without removal of the solvents or further purification.

### PREPARATION OF THE COMPOUNDS OF FORMULA IV

A substituted 4,5,6,7-tetra-hydrothieno[3,2-c]pyridine (Formula 6) in a solvent (e.g., a polar solvent, such as 5 to 15% aqueous tetrahydrofuran, methylene chloride or acetonitrile; preferably 5-15% aqueous tetrahydrofuran) and an optionally substituted phenalkyl or phenacyl halide (Formula 7) are added to a molar excess of a hydrated base (e.g., potassium carbonate, sodium carbonate, or lithium carbonate; preferably potassium carbonate at about 5 to 15%, preferably about 10% of the volume). The reaction mixture is refluxed until disappearance of the starting materials is confirmed by thin layer chromatography or the like (about 8 to 42 hours, preferably about 18 to 24 hours). The solvent is removed (e.g., by vacuum or displacement with another solvent such as toluene), the product is washed with water and then concentrated yielding the desired substituted thieno[3,2-c]pyridine derivative (Formula IV).

### Preferred Alkylation

To a solution of substituted 4,5,6,7-tetrahydrothieno[3,2-c]pyridine (Formula 6) in a solvent (e.g., tetrahydrofuran, methylene chloride or acetonitrile, preferably tetrahydrofuran) is added with stirring a molar excess of base (e.g., sodium carbonate, potassium carbonate, preferably potassium carbonate) dissolved in a polar solvent or water, preferably water. To the solution is added about 0.5 to 1.5 molar equivalents of an optionally substituted phenalkyl or phenacyl halide (Formula 7). After addition, the solution is heated to a temperature in the range of about 75°C to 125°C, preferably about 90°C to 110°C, and then cooled to the reflux temperature of the solvent used and refluxed for a period of about 1 to 5 hours, preferably about 3 hours. After reflux the solution is allowed to cool to about room temperature (e.g., 20°C to 30°C). The organic layer is separated and the aqueous layer is repeatedly extracted with a solvent (e.g., toluene, methylene chloride, ethyl acetate or *i*-propyl acetate, preferably toluene). The extracts of the aqueous layers are combined with the organic layer. The organic layer is filtered and the solvent removed yielding the desired substituted thieno[3,2-c]pyridine derivative (Formula IV).

### Phase Transfer Alkylation

Alternatively, the alkylation can be performed under phase transfer conditions, e.g., as described in GB 2,166,730, via phase transfer alkylation. The 4,5,6,7-tetrahydrothieno[3,2-c]-pyridine (Formula 6) and an optionally substituted phenalkyl or phenacyl halide (Formula 7) are dissolved in a solvent system (preferably biphasic aqueous/organic solvent system, the organic phase of which is immiscible with water, e.g., hydrocarbons such as benzene, toluene and xylene; and ethers such as isopropyl ether and diethyl ether; preferably toluene) combined with a phase transfer catalyst [e.g., a quaternary ammonium salt, such as benzyltrimethyl ammonium hydroxide, tetra-*n*-butyl ammonium hydrogen sulfate, methyltrioctylammonium chloride, benzyltriethyl ammonium chloride or tetrabutyl ammonium iodide ("TBAI"), tetrabutyl ammonium chloride or a phosphonium salt, such as tetrabutylphosphonium chloride, or a crown ether, such as 18-crown-6 or dibenzo 18-crown-6; preferably TBAI] in the presence of a base [e.g., sodium hydroxide, potassium hydroxide, lithium hydroxide, potassium carbonate, sodium carbonate, or sodium hydride; preferably sodium hydroxide] and stirred for about 24 to 72 hours, preferably about 40 hours, at room temperature. The product is separated, concentrated, and purified by the usual means.

### ALTERNATE PREPARATION OF FORMULA IV

Alternatively, the desired substituted thieno[3,2-c]pyridine derivatives (Formula IV) can be prepared by alkylation of suitably substituted derivatives of 2-(2-thienyl)ethylamine (Formula III) with an optionally substituted phenalkyl or phenacyl compound (Formula 7) to form a secondary amine followed by cyclization with formaldehyde or dimethoxymethane, according to the procedure of U.S. Patent No. 4,174,448.

### PREPARATION OF THE SALTS OF THE COMPOUNDS OF FORMULA IV

The free base of substituted thieno[3,2-c]pyridine derivatives (Formula IV) is treated with a stoichiometric amount of an appropriate acid, such as hydrochloric acid, sulfuric acid, methanesulfonic acid, HBr, or the like. Typically, the free base is dissolved in a polar organic solvent such as ethanol or methanol, and the acid is added in water, ethanol or methanol. The temperature is maintained at 0°-50°C. The resulting acid addition salt precipitates spontaneously or may be brought out of solution with a less polar solvent (such as toluene, diethylether, or ethyl acetate, preferably toluene).

The acid addition salt of substituted thieno[3,2-c]pyridine derivatives (Formula IV), such as the hydrochloride salt, may be converted to the corresponding free base by treatment with an excess of a suitable base, such as ammonia or sodium bicarbonate, typically in the presence of aqueous solvent, and at a temperature of about between 0° and 50°C. The free base form is isolated by conventional means, such as extraction with an organic solvent.

### PREFERRED COMPOUNDS

Of the compounds of Formula I, the presently preferred compound is that where R¹, R², R³ and R⁴ are hydrogen, i.e., 2,4,6-*tris*-(2-(2-thienyl)ethyl)cyanurate.

Of the compounds of Formula II, the presently preferred compound is that where R¹, R², R³ and R⁴ are hydrogen, i.e., 1,3,5-*tris*-(2-(2-thienyl)ethyl)isocyanurate.

Of the compounds of Formula IV, the presently preferred compound made by the processes of the invention is 5-(2-chlorobenzyl)-4,5,6,7-tetrahydrothieno-[3,2-c] pyridine hydrochloride, which is also known as ticlopidine hydrochloride.

### PREFERRED PROCESSES AND LAST STEPS

A preferred process for making 2,4,6-*tris*-(2-(2-thienyl)ethyl)cyanurate entails contacting 2-(2-thienyl)ethanol, an *n*-alkyllithium reagent with cyanuric chloride.

A preferred process for making 1,3,5-*tris*-(2-(2-thienyl)ethyl)isocyanurate entails contacting 2,4,6-*tris*-(2-(2-thienyl)ethyl)cyanurate with a tetraalkylphosphonium halide.

A preferred process for making the acid addition salt of ticlopidine comprises the steps of:
a. contacting 1,3,5-*tris*-(2-(2-thienyl)ethyl)isocyanurate with a base to give 2-(2-thienyl)ethylamine, and
b. converting the 2-(2-thienyl)ethylamine to the acid addition salt of ticlopidine.

A most preferred process for making the acid addition salt of ticlopidine comprises the steps of:
a. contacting 2-(2-thienyl)ethanol with *n*-butyllithium followed by cyanuric chloride to give 2,4,6-*tris*-(2-(2-thienyl)ethyl)cyanurate, and
b. converting the 2,4,6-*tris*-(2-(2-thienyl)ethyl)cyanurate to the acid addition salt of ticlopidine.

In the above-described most preferred process, further preferred is the process wherein the step of converting the 2,4,6-*tris*-(2-(2-thienyl)ethyl)cyanurate to the acid addition salt of ticlopidine comprises the steps of:
c. contacting 2,4,6-*tris*-(2-(2-thienyl)ethyl)cyanurate with tetrabutylphosphonium bromide to give 1,3,5-*tris*-(2-(2-thienyl)ethyl)isocyanurate,
d. contacting 1,3,5-*tris*-(2-(2-thienyl)ethyl)isocyanurate with a strong base to give 2-(2-thienyl)ethylamine,
e. contacting 2-(2-thienyl)ethylamine with a solution 37% aqueous formaldehyde followed by 6N hydrochloric acid to give 4,5,6,7-tetrahydrothieno[3,2-c]pyridine,
f. contacting 4,5,6,7-tetrahydrothieno[3,2-c]pyridine with sodium hydride followed by *o*-chlorobenzyl chloride to give 5-(2-chlorobenzyl)-4,5,6,7-tetrahydrothieno[3,2-c]pyridine, and
g. contacting 5-(2-chlorobenzyl)-4,5,6,7-tetrahydrothieno[3,2-c]pyridine with gaseous HCl to give 5-(2-chlorobenzyl)-4,5,6,7-tetrahydrothieno[3,2-c]pyridine hydrochloride.

### EXAMPLES

The following preparations and examples are given to enable those skilled in the art to more clearly understand and to practice the present invention. They should not be considered as limiting the scope of the invention, but merely as being illustrative and representative thereof.

### EXAMPLE 1

### PREPARATION OF 2,4,6-TRIS-(2-(2-THIENYL)ETHYL)CYANURATE

### 1A. FORMULA I WHERE R¹, R², R³ AND R⁴ ARE HYDROGEN.

*n*-Butyllithium (96.0 mmol, 60mL of 1.6 M in hexanes) was added in dropwise to a solution of 2-(2-thienyl)ethanol (12.306 g, 96.0 mmol) in dry THF (50mL) over a period of 49 min at -70°C. The solution was stirred at -70°C for 15 min then warmed to -30°C. To this solution, a solution of cyanuric chloride (5.901g, 32.0 mmol) in dry THF (50mL) was added dropwise at -20°C to -30°C over 7 min. After the addition was complete, the mixture was allowed to warm to 25°C and stirred for 18 hours. The solvent was removed under vacuum and the residue separated between H₂O (100mL) and ethyl acetate (100mL). The aqueous layer was extracted with an addition 50mL of ethyl acetate. The combined organic layers were dried (MgSO₄), filtered and concentrated under vacuum yielding a beige solid (14.437g, 98.2% yield). The solid was recrystallized from methanol yielding 2,4,6-*tris*-(2-(2-thienyl)ethyl)cyanurate as short colorless needles (13.241g, 90.0% yield). Characteristic analytical data are, m.p. 85°C; NMR (CDCl₃) δ 3.30 (t,2H,CH₂C), 4.60 (t,2H,CH₂O), 6.89-6.95 (m,2H,=CH), 7.15 (dd,1H,=CHS).

### 1B. PREPARATION OF OTHER COMPOUNDS OF FORMULA I, VARYING R¹, R², R³ AND R⁴.

By following the procedures of Example 1A and substituting 2-(2-thienyl)ethanol with the following:
2-methyl-2-(2-thienyl)ethanol,
1-methyl-2-(2-thienyl)ethanol,
1-benzyl-2-methyl-2-(2-thienyl)ethanol,
1-methyl-2-*p*-toluenyl-2-(2-thienyl)ethanol and 1-anisoyl-2-benzyl-2-(2-thienyl)ethanol;
there are obtained the following respective compounds:
2,4,6-*tris*-(2-methyl-2-(2-thienyl)ethyl)cyanurate,
2,4,6-*tris*-(1-methyl-2-(2-thienyl)ethyl)cyanurate,
2,4,6-*tris*-(1-benzyl-2-methyl-2-(2-thienyl)ethyl)cyanurate,
2,4,6-*tris*-(1-methyl-2-*p*-toluenyl-2-(2-thienyl)ethyl)cyanurate and
2,4,6-*tris*-(1-anisoyl-2-benzyl-2-(2-thienyl)ethyl)cyanurate.

### EXAMPLE 2

### PREPARATION OF 1,3,5-TRIS-(2-(2-THIENYL)ETHYL)ISOCYANURATE

### 2A. FORMULA II WHERE R¹, R², R³ AND R⁴ ARE HYDROGEN

A mixture of recrystallized 2,4,6-*tris*-(2-(2-thienyl)ethylcyanurate (1.000gm, 6.53mmol) and tetrabutylphosphonium bromide (0.679gm, 2.00mmol) was heated in a sealed tube at 125°C for 44hr. The mixture was cooled to 25°C. The residual solid was slurried in ethyl ether (50mL) then suction filtered. The precipitate was washed with fresh ethyl ether then dried under vacuum, yielding tetrabutylphosphonium bromide as a tan solid (0.611g, 90.0%). The combined mother liquors were concentrated under vacuum yielding an orange syrup (0.947g). The syrup was purified by radial chromatography on silica gel. The isocyanurate was eluted with ethyl acetate (40%) in hexanes. The eluent was concentrated under vacuum yielding 1,3,5-*tris*-(2-(2-thienyl)ethyl)isocyanurate (0.799gm, 82% yield) as a beige solid. Characteristic analytical data are m.p. 72-75°C; NMR (CDCl₃) δ 3.13 (dd,2H,CH₂C), 4.14 (dd,2H,CH₂N), 6.86 (m,1H,=CH), 6.94 (dd,1H,=CH), 7.17 (dd,1H,=CHS).

### 2B. PREPARATION OF OTHER COMPOUNDS OF FORMULA II, VARYING R¹, R², R³AND R⁴.

By following the procedures of Example 2A and substituting 2,4,6-*tris*-(2-(2-thienyl)ethylcyanurate with the following:
2,4,6-*tris*-(2-methyl-2-(2-thienyl)ethyl)cyanurate,
2,4,6-*tris*-(1-methyl-2-(2-thienyl)ethyl)cyanurate,
2,4,6-*tris*-(1-benzyl-2-methyl-2-(2-thienyl)ethyl)cyanurate,
2,4,6-*tris*-(1-methyl-2-*p*-toluenyl-2-(2-thienyl)ethyl)cyanurate and
2,4,6-*tris*-(1-anisoyl-2-benzyl-2-(2-thienyl)ethyl)cyanurate;
there are obtained the following respective compounds:
1,3,5-*tris*-(2-methyl-2-(2-thienyl)ethyl)isocyanurate,
1,3,5-*tris*-(1-methyl-2-(2-thienyl)ethyl)isocyanurate,
1,3,5-*tris*-(1-benzyl-2-methyl-2-(2-thienyl)ethyl)isocyanurate,
1,3,5-*tris*-(1-methyl-2-*p*-toluenyl-2-(2-thienyl)ethyl)isocyanurate and
1,3,5-*tris*-(1-anisoyl-2-benzyl-2-(2-thienyl)ethyl)isocyanurate.

### EXAMPLE 3

### PREPARATION OF 2-(-2-THIENYL)ETHYLAMINE

### 3A. FORMULA III WHERE R¹, R², R³ AND R⁴ ARE HYDROGEN.

A mixture of recrystallized 1,3,5-*tris*-(2-(2-thienyl)ethyl)isocyanurate (12.000gm, 78.3mmol), granular NaOH (7.833g, 0.196mol) and *n*-butanol (30mL) was refluxed for 72hr. The *n*-butanol was then recovered by distillation. The mixture was cooled and H₂O (30mL) was added. The resulting layers were separated and the aqueous layer extracted with toluene (2x10mL). The toluene was combined with the organic layer. The toluene was removed from the organic layer by distillation. The residual oil was distilled at 3mm Hg yielding 2-(2-thienyl)ethylamine (8.267g, 83% yield) as a colorless liquid. Characteristic analytical data are b.p. 84.5-86°C; NMR (CDCl₃) δ 1.14-1.27 (br,2H,NH2), 2.93-3.04 (m,4H,CH₂), 6.83 (dd,1H,=CH), 6.94 (dd,1H,=CH) and 7.15 (dd,1H,=CHS).

### 3B. PREPARATION OF OTHER COMPOUNDS OF FORMULA III

By following the procedures of Example 3A and substituting 1,3,5-*tris*-(2-(2-thienyl)ethyl)isocyanurate with the following:
1,3,5-*tris*-(2-methyl-2-(2-thienyl)ethyl)isocyanurate,
1,3,5-*tris*-(1-methyl-2-(2-thienyl)ethyl)isocyanurate,
1,3,5-*tris*-(1-benzyl-2-methyl-2-(2-thienyl)ethyl)isocyanurate,
1,3,5-*tris*-(1-methyl-2-*p*-toluenyl-2-(2-thienyl)ethyl)isocyanurate and
1,3,5-*tris*-(1-anisoyl-2-benzyl-2-(2-thienyl)ethyl)isocyanurate;
there are obtained the following respective compounds:
2-methyl-2-(2-thienyl)ethylamine,
1-methyl-2-(2-thienyl)ethylamine,
1-benzyl-2-methyl-2-(2-thienyl)ethylamine,
1-methyl-2-*p*-toluenyl-2-(2-thienyl)ethylamine and
1-anisoyl-2-benzyl-2-(2-thienyl)ethylamine.

### EXAMPLE 4

### PREPARATION OF 2-(2-THIENYL)ETHYLAMINE HYDROCHLORIDE

### 4A. FORMULA III WHERE R¹, R², R³ AND R⁴ ARE HYDROGEN.

Hydrogen chloride gas was bubbled into a solution of 2-(2-thienyl)ethylamine (15.86g, 0.125mol) in ethyl ether (450ml) until the mixture was thick with precipitate. The precipitate was filtered under vacuum, washed with ethyl ether (3x100ml) then dried under vacuum to afford a colorless solid (19.58g, 96.0%). m.p. 200-202°C

### 4B. PREPARATION OF OTHER COMPOUNDS OF FORMULA III

By following the procedures of Example 3A and substituting 2-(2-thienyl)ethylamine with the following:
2-methyl-2-(2-thienyl)ethylamine,
1-methyl-2-(2-thienyl)ethylamine,
1-benzyl-2-methyl-2-(2-thienyl)ethylamine,
1-methyl-2-*p*-toluenyl-2-(2-thienyl)ethylamine and
1-anisoyl-2-benzyl-2-(2-thienyl)ethylamine;
there are obtained the following:
2-methyl-2-(2-thienyl)ethylamine chloride,
1-methyl-2-(2-thienyl)ethylamine chloride,
1-benzyl-2-methyl-2-(2-thienyl)ethylamine chloride,
1-methyl-2-*p*-toluenyl-2-(2-thienyl)ethylamine chloride and
1-anisoyl-2-benzyl-2-(2-thienyl)ethylamine chloride.

### EXAMPLE 5

### PREPARATION OF 5-(2-CHLOROBENZYL)-4,5,6,7-TETRAHYDROTHIENO-[3,2-C]PYRIDINE HYDROCHLORIDE

### 5A. FORMULA IV WHERE R¹, R², R³, R⁴, R⁵, R⁶, R⁷ AND R⁸ ARE HYDROGEN, AND R⁹ IS CHLORO.

A solution of 37% aqueous formaldehyde (23.1kg) was added to 2-(2'-thienyl)ethylamine (35kg) dissolved in toluene (60kg). The mixture was refluxed with stirring for 4hrs. After the reflux the mixture was kept under an inert atmosphere, allowed to cool and the aqueous layer was removed. The solvent was removed from the organic layer yielding the desired formimine (a compound of Formula 5) of 2-(2-thienyl)ethylamine as a monomer [N-formylidene-2-(2-thienyl)ethylamine] or as a trimer [1,3,5-*tris*-(2-(2-thienyl)ethyl)-hexahydro-*s*-triazine]. The formimine was dissolved in tetrahydrofuran (57kg). A solution of tetrahydrofuran and hydrogen chloride (g) was prepared by adding hydrogen chloride (g) (12.3kg) to tetrahydrofuran (57kg) at -5°C to 0°C. While under an inert atmosphere, the hydrogen chloride solution in tetrahydrofuran was added to the formimine solution and stirred for 4hrs at 5°C to 0°C yielding the cyclic amine (a compound of Formula 6). An aqueous solution of potassium carbonate was prepared by dissolving potassium carbonate (76kg) in water (70kg). The aqueous potassium carbonate solution and *o*-chloro-benzylchloride (45.2kg) was then added to the mixture. The mixture was heated to 95°C to 100°C, allowed to cool and refluxed at 70°C to 75°C for 3hrs. After reflux, the reaction mixture was allowed to cool, extracted with toluene (1x55kg, 2x42.5kg), filtered, and washed with toluene yielding the desired product in toluene. The toluene was removed under reduced pressure yielding (a compound of Formula IV), 5-(2-chlorobenzyl)-4,5,6,7-tetrahydrothieno-[3,2-c]pyridine, ticlopidine free base. A methanolic solution of hydrogen chloride was prepared by adding hydrogen chloride (g) (12.5kg) to methanol (28kg). The ticlopidine free base was dissolved in toluene (44kg) and the methanolic solution of hydrogen chloride was added. Ticlopidine hydrochloride (84%) was isolated from the solution by centrifugation, washed with methanol/toluene (9:1) at 0°C and dried.

### 5B. PREPARATION OF OTHER COMPOUNDS OF FORMULA 5

By following the procedures of Example 5A and substituting 2-(2-thienyl)ethylamine with the following:
2-methyl-2-(2-thienyl)ethylamine,
1-methyl-2-(2-thienyl)ethylamine,
1-benzyl-2-methyl-2-(2-thienyl)ethylamine,
1-methyl-2-*p*-toluenyl-2-(2-thienyl)ethylamine and
1-anisoyl-2-benzyl-2-(2-thienyl)ethylamine; there are obtained the monomers and trimers of the following respective compounds:
N-formylidene-2-methyl-2-(2-thienyl)ethylamine, and 1,3,5-*tris*-(2-methyl-2-(2-thienyl)ethyl)-hexahydro-*s*-triazine,
N-formylidene-1-methyl-2-(2-thienyl)ethylamine, and 1,3,5-*tris*-(1-methyl-2-(2-thienyl)ethyl)-hexahydro-*s*-triazine,
N-formylidene-1-benzyl-2-methyl-2-(2-thienyl)-ethylamine, and 1,3,5-*tris*-(1-benzyl-2-methyl-2-(2-thienyl)ethyl)-hexahydro-*s*-triazine,
N-formylidene-1-methyl-2-*p*-toluenyl-2-(2-thienyl)-ethylamine, and 1,3,5-*tris*-(1-methyl-2-*p*-toluenyl-2-(2-thienyl)ethyl)-hexahydro-*s*-triazine, and
N-formylidene-1-anisoyl-2-benzyl-2-(2-thienyl)-ethylamine, and 1,3,5-*tris*-(1-anisoyl-2-benzyl-2-(2-thienyl)ethyl)-hexahydro-*s*-triazine.

### 5C. PREPARATION OF OTHER COMPOUNDS OF FORMULA 6

By following the procedures of Example 5A and substituting the formimine of 2-(2-thienyl)ethylamine (monomer or trimer) with the following:
N-formylidene-2-methyl-2-(2-thienyl)ethylamine, and 1,3,5-*tris*-(2-methyl-2-(2-thienyl)ethyl)-hexahydro-*s*-triazine,
N-formylidene-1-methyl-2-(2-thienyl)ethylamine, and 1,3,5-*tris*-(1-methyl-2-(2-thienyl)ethyl)-hexahydro-*s*-triazine,
N-formylidene-1-benzyl-2-methyl-2-(2-thienyl)ethylamine, and 1,3,5-*tris*-(1-benzyl-2-methyl-2-(2-thienyl)ethyl)-hexahydro-*s*-triazine,
N-formylidene-1-methyl-2-*p*-toluenyl-2-(2-thienyl)ethylamine, and 1,3,5-*tris*-(1-methyl-2-*p*-toluenyl-2-(2-thienyl)ethyl)-hexahydro-*s*-triazine, and
N-formylidene-1-anisoyl-2-benzyl-2-(2-thienyl)ethylamine, and 1,3,5-*tris*-(1-anisoyl-2-benzyl-2-(2-thienyl)ethyl)-hexahydro-*s*-triazine; there are obtained the following respective compounds:
4,5,6,7-tetrahydro-7-methyl-thieno[3,2-c]pyridine,
4,5,6,7-tetrahydro-6-methyl-thieno[3,2-c]pyridine,
4,5,6,7-tetrahydro-6-benzyl-7-methyl-thieno[3,2-c]pyridine,
4,5,6,7-tetrahydro-6-methyl-7-*p*-toluenyl-thieno[3,2-c]pyridine, and
4,5,6,7-tetrahydro-6-anisoyl-7-benzyl-thieno[3,2-c]pyridine.

### 5D. PREPARATION OF OTHER COMPOUNDS OF FORMULA IV

By following the procedures of Example 5A and substituting 4,5,6,7-tetrahydrothieno[3,2-c]pyridine with the following:
4,5,6,7-tetrahydro-7-methyl-thieno[3,2-c]pyridine,
4,5,6,7-tetrahydro-6-methyl-thieno[3,2-c]pyridine,
4,5,6,7-tetrahydro-6-benzyl-7-methyl-thieno[3,2-c]pyridine,
4,5,6,7-tetrahydro-6-methyl-7-*p*-toluenyl-thieno[3,2-c]pyridine, and
4,5,6,7-tetrahydro-6-anisoyl-7-benzyl-thieno[3,2-c]pyridine;
there are obtained the following respective compounds:
5-(2-chlorobenzyl)-4,5,6,7-tetrahydro-7-methyl-thieno[3,2-c]pyridine,
5-(2-chlorobenzyl)-4,5,6,7-tetrahydro-6-methyl-thieno[3,2-c]pyridine,
5-(2-chlorobenzyl)-4,5,6,7-tetrahydro-6-benzyl-7-methyl-thieno[3,2-c]pyridine,
5-(2-chlorobenzyl)-4,5,6,7-tetrahydro-6-methyl-7-*p*-toluenyl-thieno[3,2-c]pyridine, and
5-(2-chlorobenzyl)-4,5,6,7-tetrahydro-6-anisoyl-7-benzyl-thieno[3,2-c]pyridine.

### 5E. OTHER COMPOUNDS OF FORMULA IV

By following the procedure of Example 8A and substituting for *o*-chlorobenzyl chloride the following:
*m*-chlorobenzyl chloride,
*o*-bromobenzyl bromide,
3,4,5-trimethoxybenzyl chloride,
phenacyl bromide, and
*o*-methoxyphenacyl bromide;
there are obtained the following respective compounds:
5-(3-chlorobenzyl)-4,5,6,7-tetrahydrothieno[3,2-c]-pyridine,
5-(2-bromobenzyl)-4,5,6,7-tetrahydrothieno 3,2c]-pyridine,
5-(3,4,5-trimethoxybenzyl)-4,5,6,7-tetrahydrothieno-[3,2-c]pyridine,
5-phenacyl-4,5,6,7-tetrahydrothieno[3,2-c]pyridine, and
5-(2-methoxyphenacyl)-4,5,6,7-tetrahydrothieno-[3,2-c] pyridine.

## Claims

1. A compound of the formula: 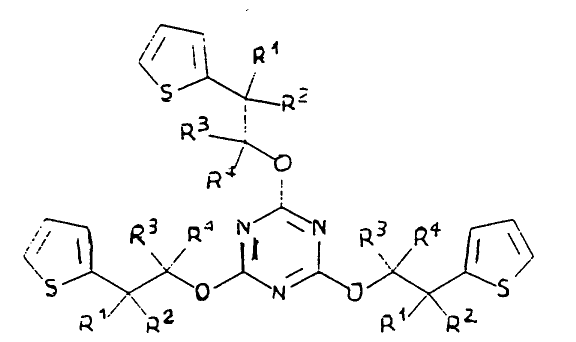 wherein:
R¹, R², R³ and R⁴ are independently hydrogen, lower alkyl of one to six carbon atoms, aryl or substituted aryl.

2. The compound of Claim 1 wherein R¹, R², R³ and R⁴ are hydrogen.

3. A compound of the formula: 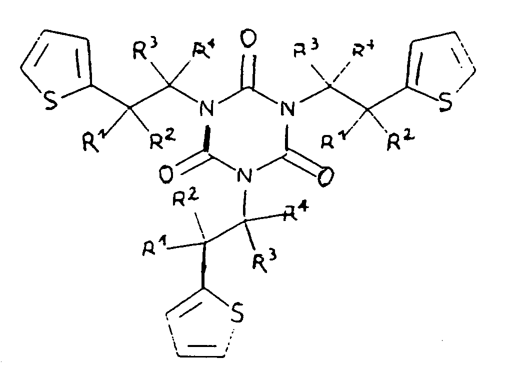 wherein:
R¹, R², R³ and R⁴ are independently hydrogen, lower alkyl of one to six carbon atoms, aryl or substituted aryl.

4. The compound of Claim 3 wherein R¹, R², R³ and R⁴ are hydrogen.

5. A process for making a compound of the formula: 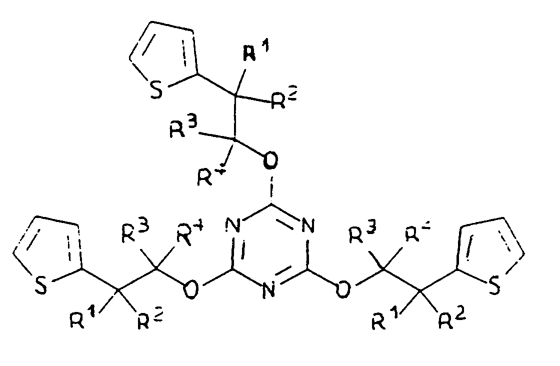 wherein:
R¹, R², R³ and R⁴ are independently hydrogen, lower alkyl of one to six carbon atoms, aryl or substituted aryl,
said process comprising:
contacting an alkyl metal salt and cyanuric halide with a compound of the formula: 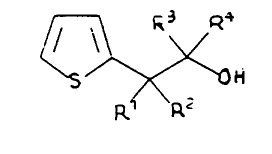
wherein:
R¹, R², R³ and R⁴ are independently hydrogen, lower alkyl of one to six carbon atoms, aryl or substituted aryl.

6. The process of Claim 5 wherein R¹, R², R³ and R⁴ are hydrogen.

7. The process of Claim 6 wherein the alkyl metal salt is alkyl lithium.

8. The process of Claim 7 wherein the alkyl lithium is n-butyllithium.

9. The process of Claim 6 wherein the cyanuric halide is cyanuric chloride.

10. A process for making a compound of the formula: 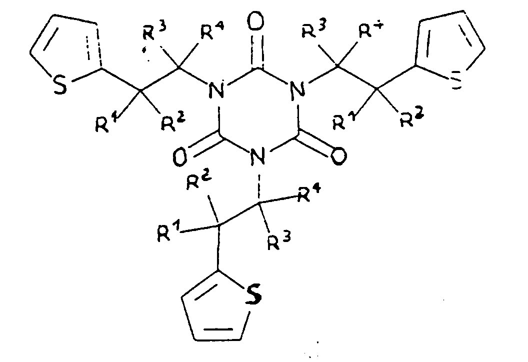 wherein:
R¹, R², R³ and R⁴ are independently hydrogen, lower alkyl of one to six carbon atoms, aryl or substituted aryl,
said process comprising:
contacting a tetraalkylphosphonium halide and a compound of the formula: 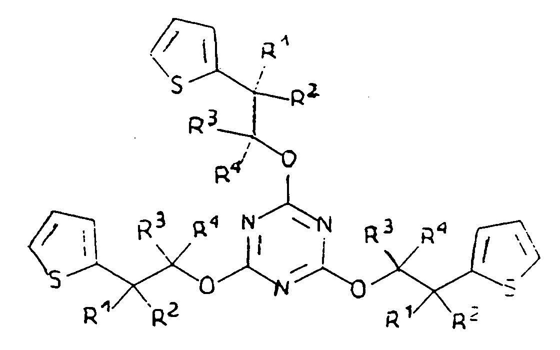
wherein:
R¹, R², R³ and R⁴ are independently hydrogen, lower alkyl of one to six carbon atoms, aryl or substituted aryl.

11. The process of Claim 10 wherein R¹, R², R³ and R⁴ are hydrogen.

12. The process of Claim 11 wherein the tetraalkylphosphonium halide is tetrabutylphosphonium bromide.

13. A process for making a compound of the formula: 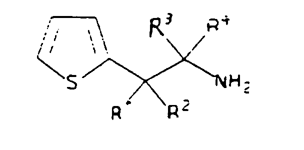 wherein:
R¹, R², R³ and R⁴ are independently hydrogen, lower alkyl of one to six carbon atoms, aryl or substituted aryl,
said process comprising:
contacting a strong base and a compound of the formula: 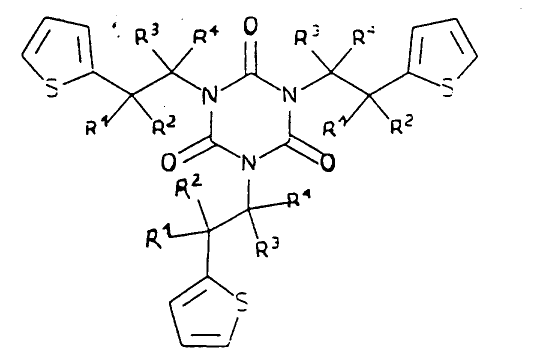
wherein:
R¹, R², R³ and R⁴ are independently hydrogen, lower alkyl of one to six carbon atoms, aryl or substituted aryl.

14. The process of Claim 13 where the strong base is sodium hydroxide.

15. The process of Claim 13 where the compound of formula: 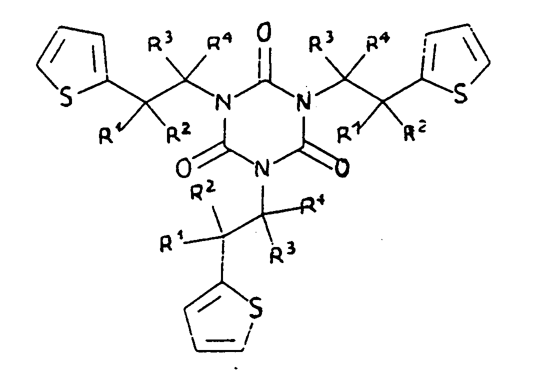 wherein:
R¹, R², R³ and R⁴ are independently hydrogen, lower alkyl of one to six carbon atoms, aryl or substituted aryl,
is prepared by contacting a tetraalkylphosphonium halide with the compound of formula: 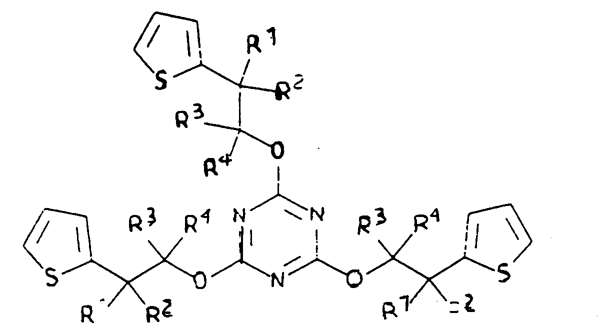 wherein:
R¹, R², R³ and R⁴ are independently hydrogen, lower alkyl of one to six carbon atoms, aryl or substituted aryl.

16. The process of Claim 15 where the tetraalkylphosphonium halide is tetrabutylphosphonium bromide.

17. A process for making a thieno[3,2-c]pyridine compound of the formula: 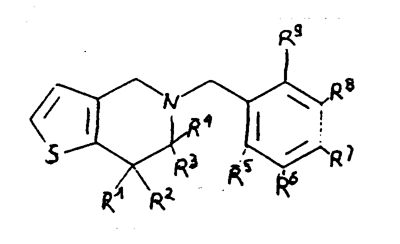 wherein:
R¹, R², R³ and R⁴ are independently hydrogen or lower alkyl of one to six carbon atoms; and
R⁵, R⁶, R⁷, R⁸ and R⁹ are independently hydrogen, lower alkyl of one to six carbon atoms, alkoxy, acyl or halo
said process comprising:
a) contacting a 2-(2-thienyl)ethylamine compound of the formula: 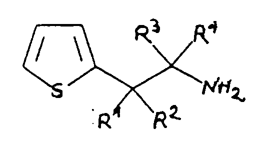 wherein:
R¹, R², R³ and R⁴ are independently hydrogen, lower alkyl of one to six carbon atoms, aryl or substituted aryl
prepared according to the process of claim 13, with an aqueous formaldehyde solution to give a formimine compound of the formula: 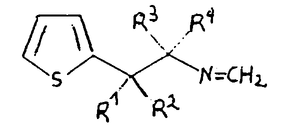
b) contacting a tetrahydrofuran solution of hydrogen chloride with said formimine to give a cyclic amine of the formula: 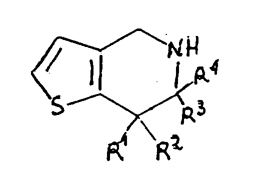 and
c) contacting a substituted aryl compound of the formula: 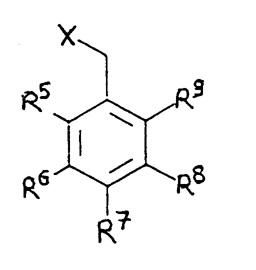 wherein:
R⁵, R⁶, R⁷, R⁸ and R⁹ are independently hydrogen, lower alkyl of one to six carbon atoms, alkoxy, acyl or halo, X is chloro or bromo;
with said cyclic amine.

18. The process of Claim 17 wherein the thieno[3,2-c]pyridine compound prepared is ticlopidine hydrochloride.

## Patentansprüche

1. Verbindung der Formel: 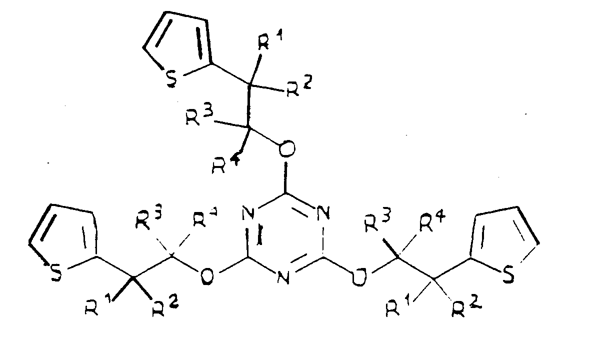 worin:
R¹, R², R³ und R⁴ unabhängig voneinander Wasserstoff, Niederalkyl mit 1 bis 6 Kohlenstoffatomen, Aryl oder substituiertes Aryl bedeuten.

2. Verbindung nach Anspruch 1, wobei R¹, R², R³ und R⁴ Wasserstoff sind.

3. Verbindung der Formel: 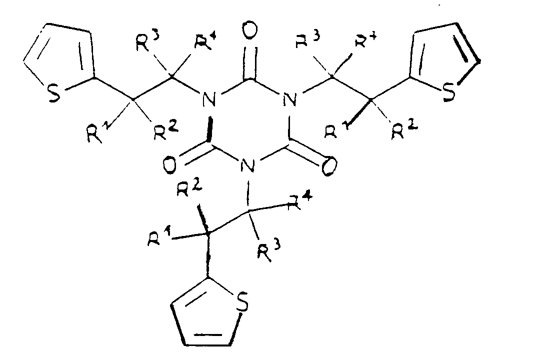 worin:
R¹, R², R³ und R⁴ unabhängig voneinander Wasserstoff, Niederalkyl mit 1 bis 6 Kohlenstoffatomen, Aryl oder substituiertes Aryl bedeuten.

4. Verbindung nach Anspruch 3, worin R¹, R², R³ und R⁴ Wasserstoff sind.

5. Verfahren zur Herstellung einer Verbindung der Formel: 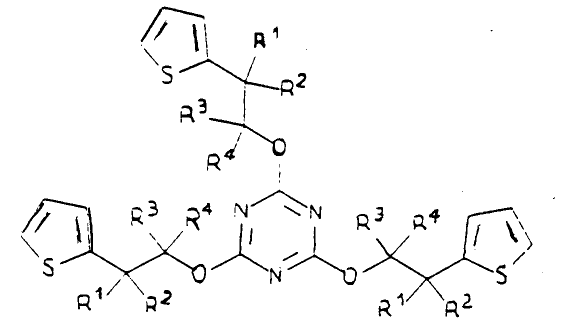 worin:
R¹, R², R³ und R⁴ unabhängig voneinander Wasserstoff, Niederalkyl mit 1 bis 6 Kohlenstoffatomen, Aryl oder substituiertes Aryl bedeuten, wobei das Verfahren das Kontaktieren eines Alkylmetallsalzes und eines Cyanursäurehalogenids mit einer Verbindung der Formel: 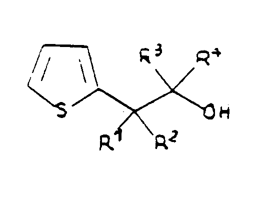 umfaßt, worin:
R¹, R², R³ und R⁴ unabhängig voneinander Wasserstoff, Niederalkyl mit 1 bis 6 Kohlenstoffatomen, Aryl oder substituiertes Aryl bedeuten.

6. Verfahren nach Anspruch 5, wobei R¹, R², R³ und R⁴ Wasserstoff sind.

7. Verfahren nach Anspruch 6, wobei das Alkylmetallsalz Alkyllithium ist.

8. Verfahren nach Anspruch 7, wobei das Alkyllithium n-Butyllithium ist.

9. Verfahren nach Anspruch 6, wobei das Cyanursäurehalogenid Cyanursäurechlorid ist.

10. Verfahren zur Herstellung einer Verbindung der Formel: 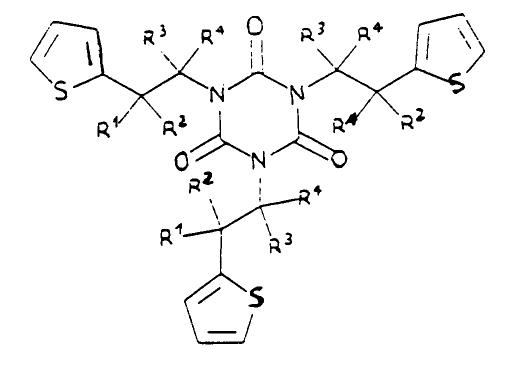 worin:
R¹, R², R³ und R⁴ unabhängig voneinander Wasserstoff, Niederalkyl mit 1 bis 6 Kohlenstoffatomen, Aryl oder substituiertes Aryl bedeuten, wobei das Verfahren das Kontaktieren eines Tetraalkylphosphoniumhalogenids und einer Verbindung der Formel: 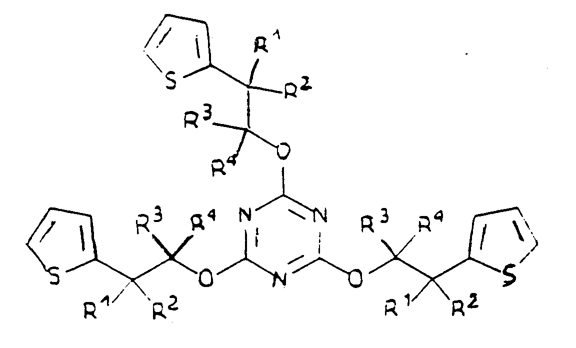 umfaßt, worin:
R¹, R², R³ und R⁴ unabhängig voneinander Wasserstoff, Niederalkyl mit 1 bis 6 Kohlenstoffatomen, Aryl oder substituiertes Aryl bedeuten.

11. Verfahren nach Anspruch 10, wobei R¹, R², R³ und R⁴ Wasserstoff sind.

12. Verfahren nach Anspruch 11, wobei das Tetraalkylphosphoniumhalogenid Tetrabutylphosphoniumbromid ist.

13. Verfahren zur Herstellung einer Verbindung der Formel: 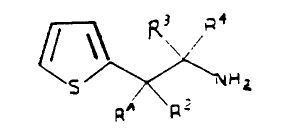 worin:
R¹, R², R³ und R⁴ unabhängig voneinander Wasserstoff, Niederalkyl mit 1 bis 6 Kohlenstoffatomen, Aryl oder substituiertes Aryl bedeuten, wobei das Vefahren das Kontaktieren einer starken Base und einer Verbindung der Formel: 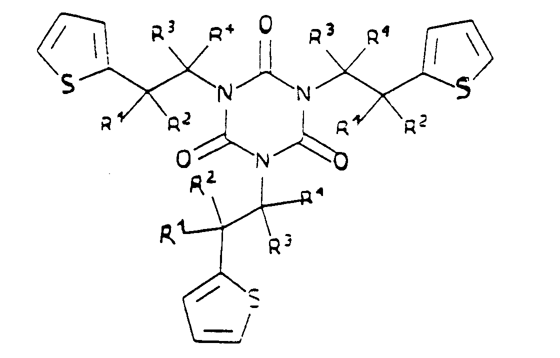 umfaßt, worin:
R¹, R^{2,} R³ und R⁴ unabhängig voneinander Wasserstoff, Niederalkyl mit 1 bis 6 Kohlenstoffatomen, Aryl oder substituiertes Aryl bedeuten.

14. Verfahren nach Anspruch 13, wobei die starke Base Natriumhydroxid ist.

15. Verfahren nach Anspruch 13, wobei die Verbindung der Formel: 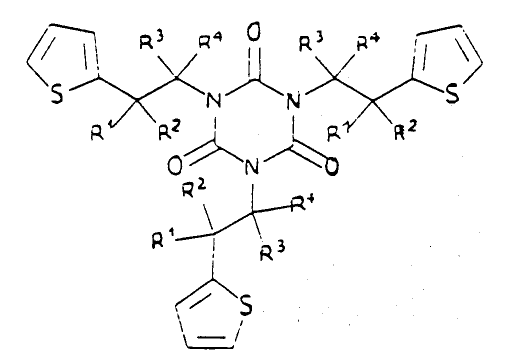 worin:
R¹, R², R³ und R⁴ unabhängig voneinander Wasserstoff, Niederalkyl mit 1 bis 6 Kohlenstoffatomen, Aryl oder substituiertes Aryl bedeuten, hergestellt wird durch Kontaktieren eines Tetraalkylphosphoniumhalogenids mit der Verbindung der Formel: 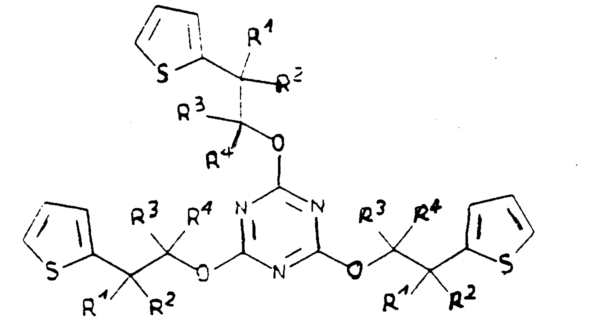 worin:
R¹, R², R³ und R⁴ unabhängig voneinander Wasserstoff, Niederalkyl mit 1 bis 6 Kohlenstoffatomen, Aryl oder substituiertes Aryl bedeuten.

16. Verfahren nach Anspruch 15, wobei das Tetraalkylphosphoniumhalogenid Tetrabutylphosphoniumbromid ist.

17. Verfahren zur Herstellung einer Thieno[3,2-c]pyridinverbindung der Formel: 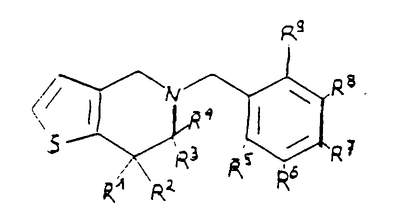 worin:
R¹, R², R³ und R⁴ unabhängig voneinander Wasserstoff oder Niederalkyl mit 1 bis 6 Kohlenstoffatomen bedeuten;
und
R⁵, R⁶, R⁷, R⁸ und R⁹ unabhängig voneinander Wasserstoff, Niederalkyl mit 1 bis 6 Kohlenstoffatomen, Alkoxy, Acyl oder Halogen bedeuten,
wobei das Verfahren
a) das Kontaktieren einer 2-(2-Thienyl)ethylaminverbindung der Formel: 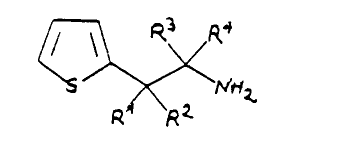 worin:
R¹, R², R³ und R⁴ unabhängig voneinander Wasserstoff, Niederalkyl mit 1 bis 6 Kohlenstoffatomen, Aryl oder substituiertes Aryl bedeuten, welche gemäß dem Verfahren nach Anspruch 13 hergestellt worden ist, mit einer wäßrigen Formaldehydlösung zur Erzielung einer Formiminverbindung der Formel: 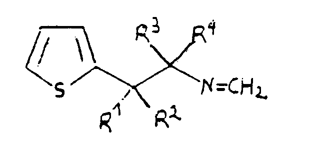
b) das Kontaktieren einer Tetrahydrofuranlösung von Wasserstoffchlorid mit dem Formimin zur Erzielung eines cyclischen Amins der Formel: 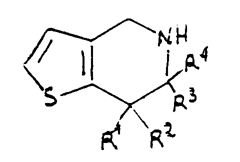 und
c) das Kontaktieren einer substituierten Arylverbindung der Formel: 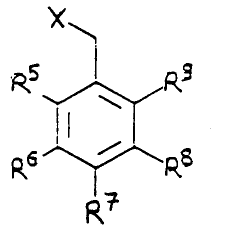 worin:
R⁵, R⁶, R⁷, R⁸ und R⁹ unabhängig voneinander Wasserstoff, Niederalkyl mit 1 bis 6 Kohlenstoffatomen, Alkoxy, Acyl oder Halogen bedeuten und X Chlor oder Brom ist;
mit dem cyclischen Amin umfaßt.

18. Verfahren nach Anspruch 17, wobei die hergestellte Thieno[3,2-c]pyridinverbindung Ticlopidinhydrochlorid ist.

## Revendications

1. Composé de formule 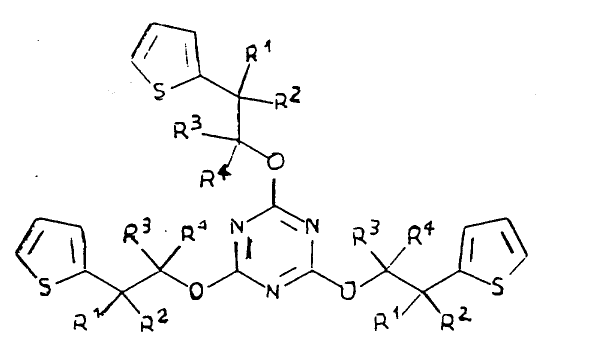 dans laquelle :
R¹, R², R³ et R⁴ sont indépendamment un atome d' hydrogène, un groupe alkyle inférieur de un à six atomes de carbone, un groupe aryle ou un groupe aryle substitué.

2. Composé selon la revendication 1, où R¹, R², R³ et R⁴ sont un atome d'hydrogène.

3. Composé de formule 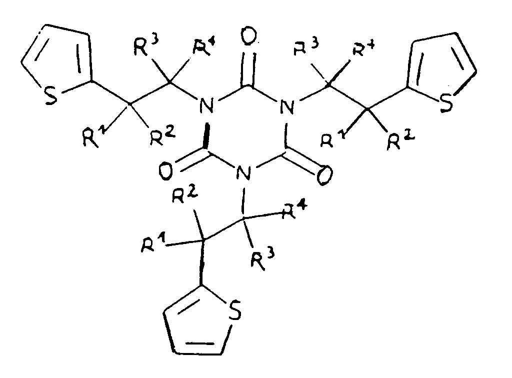 dans laquelle :
R¹, R², R³ et R⁴ sont indépendamment un atome d'hydrogène, un groupe alkyle inférieur de un à six atomes de carbone, un groupe aryle ou un groupe aryle substitué.

4. Composé selon la revendication 3, où R¹, R², R³ et R⁴ sont un atome d'hydrogène.

5. Procédé pour préparer un composé de formule : 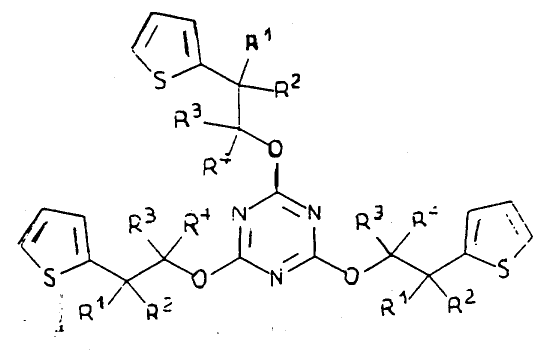 dans laquelle
R¹, R², R³ et R⁴ sont indépendamment un atome d' hydrogène, un groupe alkyle inférieur de un à six atomes de carbone, un groupe aryle ou un groupe aryle substitué,
ledit procédé comprenant :
la mise en contact d'un sel alkyl-métallique et d'un halogénure de cyanuryle avec un composé de formule : 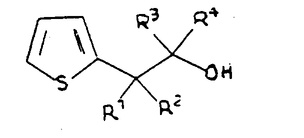 dans laquelle :
R¹, R², R³ et R⁴ sont indépendamment un atome d' hydrogène, un groupe alkyle inférieur de un à six atomes de carbone, un groupe aryle ou un groupe aryle substitué.

6. Procédé selon la revendication 5, où R¹, R², R³ et R⁴ sont un atome d'hydrogène.

7. Procédé selon la revendication 6, où le sel alkyl-métallique est un alkyl-lithium.

8. Procédé selon la revendication 7, où l'alkyl-lithium est le n-butyllithium.

9. Procédé selon la revendication 6, où l'halogénure de cyanuryle est le chlorure de cyanuryle.

10. Procédé pour préparer un composé de formule : 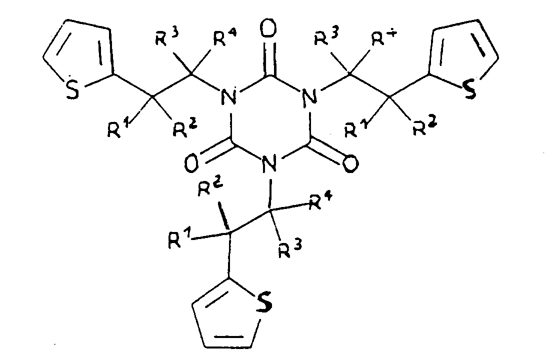 dans laquelle :
R¹, R², R³ et R⁴ sont indépendamment un atome d'hydrogène, un groupe alkyle inférieur de un à six atomes de carbone, un groupe aryle ou un groupe aryle substitué,
ledit procédé comprenant :
la mise en contact d'un halogénure de tétraalkylphosphonium et d'un compose de formule : 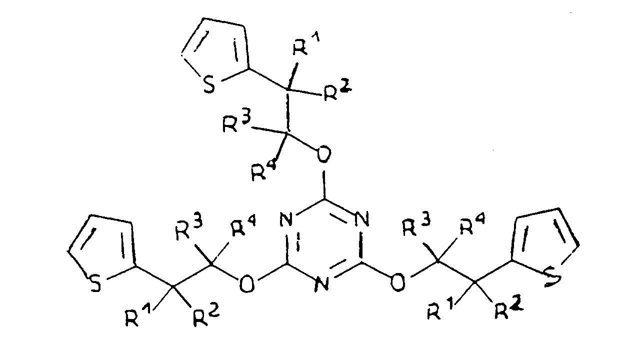 dans laquelle
R¹, R², R³ et R⁴ sont indépendamment un atome d' hydrogène, un groupe alkyle inférieur de un à six atomes de carbone, un groupe aryle ou un groupe aryle substitué.

11. Procédé selon la revendication 10, où R¹, R², R³ et R⁴ sont un atome d'hydrogène.

12. Procédé selon la revendication 11, où l'halogénure de tétraalkylphosphonium est le bromure de tétrabutylphosphonium.

13. Procédé pour préparer un composé de formule : 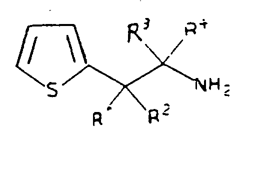 dans laquelle :
R¹, R², R³ et R⁴ sont indépendamment un atome d' hydrogène, un groupe alkyle inférieur de un à six atomes de carbone, un groupe aryle ou un groupe aryle substitué,
ledit procédé comprenant :
la mise en contact d'une base forte et d'un composé de formule : 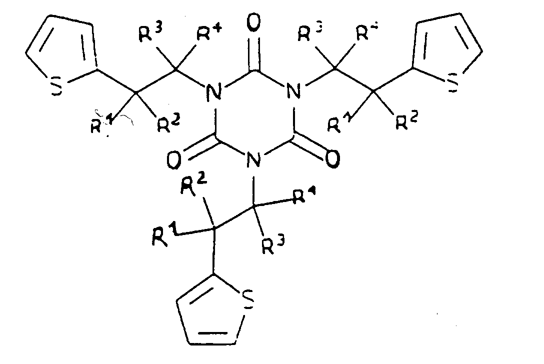 dans laquelle :
R¹, R², R³ et R⁴ sont indépendamment un atome d'hydrogène, un groupe alkyle inférieur de un à six atomes de carbone, un groupe aryle ou un groupe aryle substitué.

14. Procédé selon la revendication 13, où la base forte est l'hydroxyde de sodium.

15. Procédé selon la revendication 13, où le composé de formule : 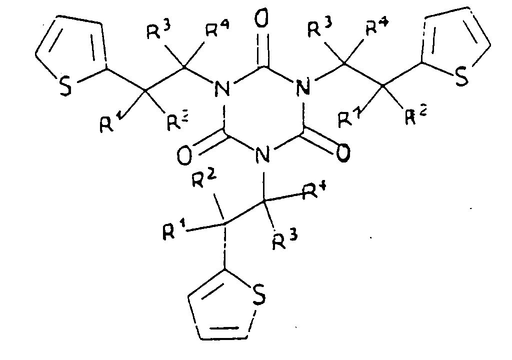 dans laquelle :
R¹, R², R³ et R⁴ sont indépendamment un atome d' hydrogène, un groupe alkyle inférieur de un à six atomes de carbone, un groupe aryle ou un groupe aryle substitué,
est préparé par mise en contact d'un halogénure de tétraalkylphosphonium avec un composé de formule : 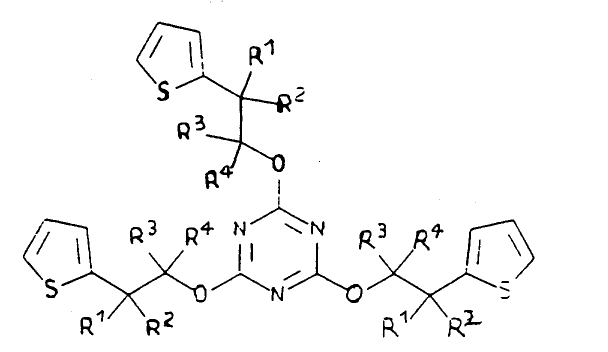 dans laquelle :
R¹, R², R³ et R⁴ sont indépendamment un atome d' hydrogène, un groupe alkyle inférieur de un à six atomes de carbone, un groupe aryle ou un groupe aryle substitué.

16. Procédé selon la revendication 15, où l'halogénure de tétraalkylphosphonium est le bromure de tétrabutylphosphonium.

17. Procédé pour préparer une thiéno[3,2-c]pyridine de formule : 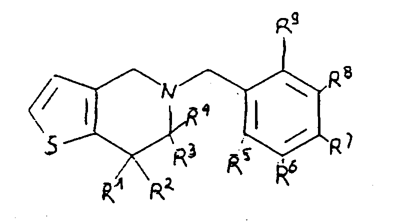 dans laquelle :
R¹, R², R³ et R⁴ sont indépendamment un atome d'hydrogène ou un groupe alkyle inférieur de un à six atomes de carbone ; et
R⁵, R⁶, R⁷, R⁸ et R⁹ sont indépendamment un atome d'hydrogène, un groupe alkyle inférieur de un à six atomes de carbone, un groupe alcoxy, un groupe acyle ou un groupe halogéno,
ledit procédé comprenant
a) la mise en contact d'une 2-(2-thiényl)éthylamine de formule : 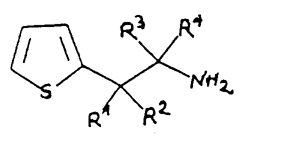 dans laquelle :
R¹, R², R³ et R⁴ sont indépendamment un atome d' hydrogène, un groupe alkyle inférieur de un à six atomes de carbone, un groupe aryle ou un groupe aryle substitué,
préparée selon le procédé de la revendication 13, avec une solution aqueuse de formaldéhyde, pour former une formimine de formule : 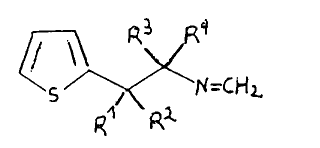
b) la mise en contact d'une solution de chlorure d'hydrogène dans le tétrahydrofuranne avec ladite formimine, pour former une amine cyclique de formule : 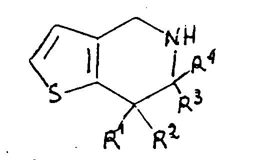 et
c) la mise en contact d'un composé arylique substitué de formule : 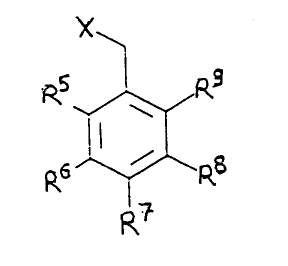 dans laquelle :
R⁵, R⁶, R⁷, R⁸ et R⁹ sont indépendamment un atome d'hydrogène, un groupe alkyle inférieur de un à six atomes de carbone, un groupe alcoxy, un groupe acyle ou un atome d'halogène et X est chloro ou bromo ;
avec ladite amine cyclique.

18. Procédé selon la revendication 17, où la thiéno[3,2-c] pyridine préparée est le chlorhydrate de ticlopidine.
